(19) Europäisches Patentamt
European Patent Office
Office européen des brevets

(11) Publication number : 0 301 861 B1

(12) **EUROPEAN PATENT SPECIFICATION**

(45) Date of publication of patent specification : **01.04.92 Bulletin 92/14**

(21) Application number : **88306963.5**

(22) Date of filing : **28.07.88**

(51) Int. Cl.$^5$ : **A61K 31/165,** A61K 31/16, C07C 259/00, C07D 213/56, C07D 213/61, C07D 213/63, C07D 215/12, C07D 215/18, C07D 215/20, C07D 217/18, C07D 217/22

(54) **Cell proliferation inhibitors.**

(30) Priority : **29.07.87 JP 189143/87**

(43) Date of publication of application : **01.02.89 Bulletin 89/05**

(45) Publication of the grant of the patent : **01.04.92 Bulletin 92/14**

(84) Designated Contracting States : **AT BE CH DE ES FR GB GR IT LI LU NL SE**

(56) References cited :
**EP-A- 0 092 136**
**EP-A- 0 171 251**
**EP-A- 0 232 089**
**EP-A- 0 234 729**
**GB-A- 1 444 492**
**US-A- 4 514 420**

(73) Proprietor : **TAKEDA CHEMICAL INDUSTRIES, LTD.**
**3-6, Doshomachi 2-chome Chuo-ku**
**Osaka (JP)**

(72) Inventor : **Hashimoto, Naoto**
**4-15, Tsukumodai 4-chome**
**Suita Osaka 565 (JP)**
Inventor : **Kato, Kaneyoshi**
**H-407, 17 Shin-ashiyakami**
**Suita Osaka 565 (JP)**
Inventor : **Kozai, Yoshio**
**9-5, Seifuso 1-chome**
**Toyonaka Osaka 560 (JP)**

(74) Representative : **Lewin, John Harvey et al**
**Elkington and Fife Prospect House 8**
**Pembroke Road**
**Sevenoaks, Kent TN13 1XR (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a cell-proliferation inhibitor containing, as an effective ingredient, a hydroxamic acid derivative, which is effective in treatment and prevention of cancer and autoimmune diseases.

Cell proliferation is an essential function for growth of organisms and for maintenance of their lives. In higher animals, most tissues and organs have their own proliferation mechanizms which are controlled by various regulating systems. Recently, many substances that control cell proliferation, i.e. "cell growth factors", have been isolated, purified, and clarified to play important roles in construction and maintenance of individual bodies. On the other hand, there are many reports that abnormal proliferation, especially out-of-control, unlimited proliferation may be involved in various diseases. Cancer is a representative example. It has been clarified that tumor cells release angiogenic substances to maintain their proliferation so that peripheral and inside regions of the cancer tissues are neovascularized; it has been almost clarified that the factors (angiogenic factors) are very effective for proliferation of vascular endothelial cells. Neovascularization is also observed in the pathological conditions such as chronic inflammation, diabetic retinopathy, psoriasis, rheumatic arthritis, etc., and has been suggested to be involved in progress of these diseases.

It has been also known that growth factors are involved in activation of immunocytes, particularly of lymphocytes, and therefore overproduction or overresponse to the growth factors may be some aggravating factors in autoimmune diseases and allergic diseases. Therefore, if a drug that can selectively inhibit the growth factors, involved in these diseases or suppress the response of the factors, could be developed, the drug would be an effective measure for prevention and treatment of these diseases and also for suppressing the rejection in transplantation.

This invention provides a cell-proliferation inhibitor and a hydroxamic acid derivative, having inhibitory activity of cell proliferation.

This invention relates to

1) Use of compound of the formula (I) for the manufacture of a pharmaceutical composition for inhibiting cell proliferation in mammals :

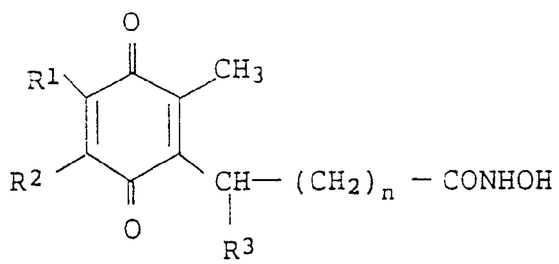

(I)

wherein, $R^1$ and $R^2$ are the same or different, representing methyl or methoxy groups, or form -CH=CH-CH=CH- by binding to each other; $R^3$ is an aromatic group or a heterocyclic group (excluding nitrogen-containing heterocyclic groups) which may be substituted; and n is an integer of 2 to 8.

2) A compound of the formula:

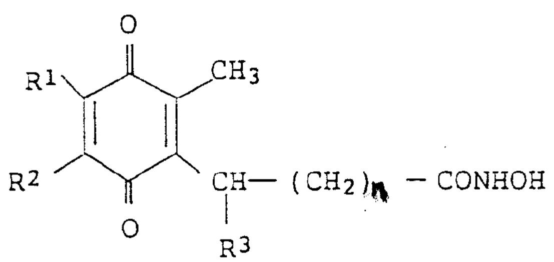

(I')

wherein $R^1$, $R^2$ and $R^3$ and n have the meanings given above, but excluding compounds in which $R^1$ and $R^2$ are each methyl, $R^3$ is optionally substituted phenyl and n is 4.

The cell-proliferation inhibitor is the pharmaceutical composition shown above.

The aromatic groups represented by $R^3$ in the general formula (I) described above include aryl groups such as phenyl, naphthyl, and indanyl (4-indanyl, 5-indanyl) groups, and the heterocyclic groups include 5- or 6-membered monocyclic or bicyclic groups containing at least one of oxygen or sulfur atoms as the ring-constituting

atoms, such as thienyl (2-thienyl, 3-thienyl) and furyl (2-furyl, 3-furyl). Among these, phenyl and thienyl groups are desirable. These aromatic and heterocyclic groups may have one to five, desirably one to three, substituents at any positions, and the substituents include halogen atoms such as fluorine, chlorine, and bromine atoms, alkyl groups having one to three carbon atoms such as methyl, ethyl, and propyl groups, and alkoxy groups having one to three carbon atoms such as methoxy, ethoxy, and isopropoxy groups.

As the $R^3$, phenyl, 4-fluorophenyl, 4-methoxyphenyl, 5-methyl-2-thienyl are preferable. n is preferably an integer of 4 to 6 and the most preferably 5 or 6.

The compound of the formula (I) can be prepared by reacting a compound of the formula:

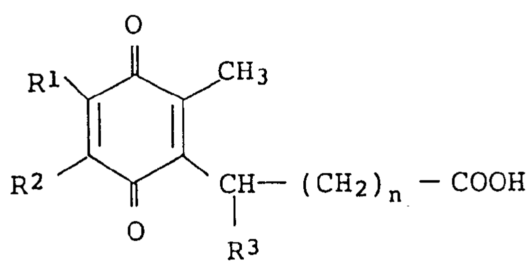

(II)

wherein each symbol has the meaning given above, with a carboxylic acid-activator to give a derivative which is reactive at the carboxylic group, followed by the reaction with hydroxylamine.

The carboxylic acid-activation include thionyl chloride, phosphorus pentachloride, chloroformic acid esters (methyl chloroformate, ethyl chloroformate), oxalyl chloride and carbodiimides (N,N-dicyclohexylcarbodiimide (DCC)), and a carbodiimide and p-nitrophenol or hydroxysuccinimide may be combined. The reaction is carried out usually in the presence of a halogenated hydrocarbon such as methylene chloride and chloroform, an ether (such as tetrahydrofuran (THF), dioxane, dimethylether, diethylether, and isopropylether), N,N-dimethylformamide, or a mixture thereof. The reaction temperature is usually -10°C to 50°C.

When thionyl chloride, oxalyl chloride, or phosphoruspentachloride is used as the carboxylic acid-activator in the reaction, the reactive derivative obtained is an acid halide; when a chloroformic ester is used as the carboxylic acid-activator, the reactive derivative obtained is a mixed acid anhydride; and when a carbodiimide is used as the carboxylic acid-activator, the reactive derivative obtained is an active ester.

The reaction between the derivative which is reactive at the carboxyl group and hydroxylamine is carried out, when the reactive derivative is an acid halide, in a solvent such as dichloromethane, tetrahydrofuran, and acetone, in the presence of an acid-binding agent (pyridine, triethylamine, potassium carbonate, sodium carbonate, potassium hydrogencarbonate, sodium hydrogencarbonate, etc.) under anhydrous or hydrous conditions. The reaction temperature is -10°C to 30°C. When the reactive derivative is an active ester or a mixed acid anhydride, the reaction can be carried out in the same solvent as used in the reaction of the compound (II) with a carboxylic acid activator. The reaction temperature in this case is usually 0 to 30°C, and the reaction time is 1 to 5 hours.

The hydroxamic acid derivatives (I) thus produced can be isolated by the per se known methods of separation and purification (e.g. chromatography, crystallization).

The hydroxamic acid derivatives (I) have an asymetric carbon atom at the alpha (α) carbon on the side chain of the quinone nucleus of the structure. This means that the compounds (I) include optically active compounds and racemic compounds.

The compounds (I) can inhibit the proliferation of various cells (endothelial cells, lymphocytes, tumor cells, etc.), and therefore they can inhibit neovascularization, immunity, and proliferation of tumor cells. In addition, the compounds have extremely low toxicity, rarely giving rise to side effects. Therefore the compounds (I) are useful for treatment and prevention of the diseases such as diabetic retinopathy, psoriasis, rheumatism, chronic inflammation, autoimmune diseases, and cancer in mammals (mouse, rat, rabbit, monkey, horse, human, etc.). The compounds are also useful to suppress the rejection in transplantation.

In addition the compounds (I) can improve the metabolism of poly-unsaturated fatty acids (linoleic acid, γ-linolenic acid, α-linolenic acid, arachidonic acid, dihomo-γ-linolenic acid, eicosapentaenoic acid), particularly they inhibit the production of oxigenenated fatty acids (anti-oxidant activity) and metabolisms of the 5-lipoxygenase system (e.g. leukotrienes, 5-hydroxyeicosatetraenoic acid, 5-peroxyeicosatetraenoic acid, lipoxins, $LTB_4$); the compounds are expected to be effective in treatment and prevention of bronchial asthma, inflammation, immediate hypersensitivity, arteriosclerosis, atherosclerosis, fatty liver, hepatitis, cirrhosis of the liver, hypersensitivity pneumonia; the compounds are useful as drugs such as those for asthma, antiallergic agents, cerebrocardiovascular system-improving agents, coronary sclerosis-preventive-agents, immunoregulatory agents,

prostaglandin-thromboxane metabolism-improving agents, therapeutics for fatty liver, hepatitis, cirrhosis of the liver, and hypersensitivity pneumonia.

The cell-proliferation inhibitor of the present invention is pharmaceutical composition containing a compound of the formula (I) and pharmaceutically acceptable carrier, vehicle or diluent therefor. The pharmaceutical composition may be tablets, capsules (e.g. soft capsules, microcapsules), granules, powders, liquid preparations, injections or suppositories. These pharmaceutical preparations can be prepared by a per se known conventional method.

Though the dose level varies according to the subjects, route of administration, symptoms, etc., for example, for oral or parenteral administration to an adult patient, the daily dose as compound (I) is about 0.1 mg/kg to 40 mg/kg body weight, desirably about 0.2 mg/kg to 20 mg/kg body weight.

Each of the compounds (I) has a bulky group at the alpha ($\alpha$) carbon atom, and this characteristic structure makes it difficult to be inactivated in in vivo metabolism; therefore the effective blood level of the drug can be maintained for a longer period and the drug has an excellent effect at a lower dose, as compared with the known quinone compounds.

Among the compounds of the formula (I), the compounds of the formula (I') are novel compounds.

The compounds (II) can be prepared for example by the method described in the gazette of Japanese Unexamined Patent Publication No. 44840/1986.

Because the novel hydroxamic acid derivatives in this invention can inhibit cell proliferation, neovascularization, and proliferation of tumor cells, and suppress immunity, the derivatives can be used not only as anticancer agents but also for suppression of the rejection in transplantation.

Example 1

7-(4-Methoxyphenyl)-7-(3,5,6-trimethyl-1,4-benzoquinon-2-yl)-heptanoic acid (1.3 g, 3.3 mmol) was dissolved in dichloromethane (20 ml), to which oxalyl chloride (1 ml) was added at room temperature. The reaction mixture was stirred at 50°C for 1 hour, and the solvent was evaporated under reduced pressure. The resultant residue was dissolved in THF (5 ml), which was added dropwise at room temperature to a mixture of hydroxylamine hydrochloride (1 g, 14 mmol) in THF (10 ml) and saturated solution of aqueous sodium hydrogencarbonate (10 ml). After being stirred at room temperature for 1 hour, ethyl acetate (20 ml) was added to the reaction mixture. The organic layer was washed with water, dried, and concentrated under reduced pressure, to give 7-(4-methoxyphenyl)-7-(3,5,6-trimethyl-1,4-benzoquinon-2-yl)-heptanohydroxamic acid (0.6 g, 42%). The physical properties are shown in the column of Compound No.8 in Table 1. In the similar manner, Compounds Nos. 1, 4, 10, 11, 12, 13, 14, 15, 16, 17, 19, 20, 21, 22, 23, 24, 25, and 26 were synthesized.

Example 2

7-(4-Fluorophenyl)-7-(3,5,6-trimethyl-1,4-benzoquinon-2-yl)-heptanoic acid (0.8 g, 2.2 mmol) was dissolved in dichloromethane (20 ml), to which oxalyl chloride (0.5 ml) was added at room temperature. The reaction mixture was stirred at 50°C for 1 hour, and the solvent was evaporated under reduced pressure. The resultant residue was dissolved in THF (5 ml), which was added dropwise at room temperature to a mixture of hydroxylamine hydrochloride (0.5 g, 7 mmol) in THF (10 ml) and saturated solution of aqueous sodium hydrogencarbonate (10 ml). After being stirred at room temperature for 1 hour, ethyl acetate (20 ml) was added to the reaction mixture. The organic layer was washed with water, dried, and concentrated under reduced pressure, and the resultant residue was recrystallized from isopropylether, to give 7-(4-fluorophenyl)-7-(3,5,6-trimethyl-1,4-benzoquinon-2-yl)-heptanohydroxamic acid (0.7 g, 85%). The physical properties are shown in the column of Compound No.6 in Table 1. In the similar manner, Compounds Nos. 2, 3, 5, 7, 9, and 18 were synthesized.

Table 1 Compounds (I)

Table 1

| Compound No. | $R^1$ | $R^2$ | $R^3$ | n | molecular formula boiling point (°C) | NMR spectra (in $CDCl_3$), δ(ppm) |
|---|---|---|---|---|---|---|
| 1 | Me | Me | 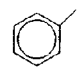 | 2 | $C_{19}H_{21}NO_4$ oil | 1.96(6H,s),2.02(3H,s),2.0-2.6(4H,m), 4.31(1H,t,7Hz),7.24(5H,m) |
| 2 | Me | Me | 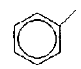 | 4 | $C_{21}H_{25}NO_4$ 59-60 | 1.1-1.8(4H,m),2.0-2.4(4H,m),1.97(6H,s), 2.03(3H,s),4.26(1H,t,7Hz),7.27(5H,m) |
| 3 | Me | Me | 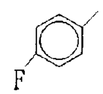 | 4 | $C_{21}H_{24}NO_4F$ 98-99 | 1.1-1.8(4H,m),2.0-2.4(4H,m),1.96(6H,s), 2.03(3H,s), 4.21(1H,t,7Hz),6.94(2H,m), 7.21(2H,m) |
| 4 | Me | Me | 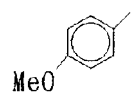 | 4 | $C_{22}H_{27}NO_5$ oil | 1.0-1.8(4H,m),2.0-2.4(4H,m),1.97(6H,s), 2.04(3H,s),3.75(3H,s),4.19(1H,t,7Hz), 6.80(2H,d,8Hz),7.19(2H,d,8Hz) |
| 5 | Me | Me | 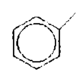 | 5 | $C_{22}H_{27}NO_4$ 60-61 | 1.1-1.8(4H,m),2.0-2.4(4H,m),1.97(6H,s), 2.02(3H,s),4.27(1H,t,7Hz),7.22(5H,m) |
| 6 | Me | Me | 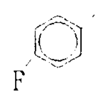 | 5 | $C_{22}H_{26}NO_4F$ 97-98 | 1.1-1.8(6H,m),2.0-2.4(4H,m),1.96(6H,s), 2.04(3H,s),4.21(1H,t,7Hz),6.94(2H,m), 7.26(2H,m) |

Table 1 (continued)

| Compound No. | $R^1$ | $R^2$ | $R^3$ | n | molecular formula boiling point (°C) | NMR spectra (in $CDCl_3$), δ(ppm) |
|---|---|---|---|---|---|---|
| 7 | Me | Me | 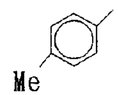 | 5 | $C_{23}H_{29}NO_4$ 69-70 | 1.1-1.8(6H,m),2.0-2.4(4H,m),1.96(6H,s), 2.02(3H,s),2.27(3H,s),4.21(1H,t,7Hz), 7.09(4H,m) |
| 8 | Me | Me | 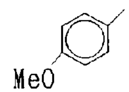 | 5 | $C_{23}H_{29}NO_5$ oil | 1.1-1.8(6H,m),2.0-2.4(4H,m),1.97(6H,s), 2.04(3H,s),3.75(3H,s),4.18(1H,t,7Hz), 6.78(2H,d,8Hz),7.20(2H,d,8Hz) |
| 9 | Me | Me | 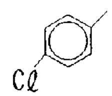 | 5 | $C_{22}H_{26}NO_4Cl$ 58-59 | 1.1-1.8(6H,m),2.0-2.4(4H,m),2.00(6H,s), 2.05(3H,s), 4.20(1H,t,7Hz),7.23(4H,m) |
| 10 | Me | Me | 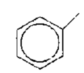 | 6 | $C_{23}H_{29}NO_4$ oil | 1.1-1.8(8H,m),2.0-2.4(4H,m),1.99(6H,s), 2.04(3H,s),4.03(1H,t,7Hz),7.27(5H,m) |
| 11 | 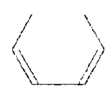 | | 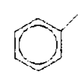 | 3 | $C_{22}H_{21}NO_4$ oil | 1.4-1.8(2H,m),2.0-2.4(4H,m),2.17(3H,s), 4.49(1H,t,7Hz),7.26(5H,m),7.66(2H,m), 8.09(2H,m) |
| 12 | 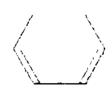 | | 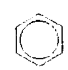 | 4 | $C_{23}H_{23}NO_4$ oil | 1.1-1.8(4H,m),2.0-2.4(4H,m),4.49(1H,t, 7Hz),7.28(5H,m),7.70(2H,m),8.03(2H,m) |

Table 1 (continued)

| Compound No. | $R^1$ | $R^2$ | $R^3$ | n | molecular formula boiling point (°C) | NMR spectra (in $CDCl_3$), δ(ppm) |
|---|---|---|---|---|---|---|
| 13 | 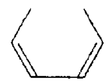 | | 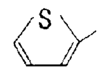 | 4 | $C_{21}H_{21}NO_4S$ oil | 1.1-1.8(4H,m),2.0-2.4(4H,m),2.20(3H,m), 4.62(1H,t,7Hz),6.88(2H,m),7.10(1H,m), 7.66(2H,m),8.03(2H,m) |
| 14 | 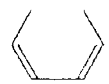 | | 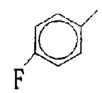 | 5 | $C_{24}H_{24}NO_4F$ oil | 1.1-1.8(6H,m),2.0-2.4(4H,m),2.17(3H,s), 4.37(1H,t,7Hz),6.95(2H,m),7.27(2H,m), 7.65(2H,m),7.99(2H,m) |
| 15 | 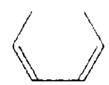 | | 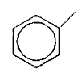 | 6 | $C_{25}H_{27}NO_4$ oil | 1.1-1.8(8H,m),2.0-2.4(4H,m),2.19(3H,s), 4.40(1H,t,7Hz),7.24(5H,m) |
| 16 | MeO | MeO | 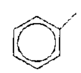 | 5 | $C_{22}H_{27}NO_6$ oil | 1.1-1.8(6H,m),2.0-2.4(4H,m),2.01(3H,s), 3.95(6H,s),4.27(1H,t,7Hz),7.23(5H,m) |
| 17 | MeO | MeO | 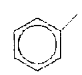 | 3 | $C_{20}H_{23}NO_6$ oil | 1.4-1.8(2H,m),2.0-2.4(4H,m),2.01(3H,s), 3.94(6H,s),4.26(1H,t,7Hz),7.23(5H,m) |
| 18 | 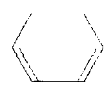 | | 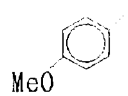 | 4 | $C_{22}H_{27}NO_5$ 115-116 | 1.1-1.8(4H,m),2.0-2.4(4H,m),2.19(3H,s), 3.74(3H,s),4.36(1H,t,7Hz),6.80(2H,d,8 Hz),7.28(2H,d,8Hz),7.64(2H,m),8.01(2H,m) |

Table 1 (continued)

| Compound No. | $R^1$ | $R^2$ | $R^3$ | n | molecular formula boiling point (°C) | NMR spectra (in $CDCl_3$), δ(ppm) |
|---|---|---|---|---|---|---|
| 19 | 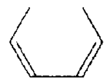 | | 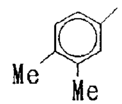 | 5 | $C_{26}H_{29}NO_4$ oil | 1.1-1.8(6H,m),1.9-2.5(4H,m),2.19(3H,s),4.37(1H,t,7Hz),7.03(3H,m),7.64(2H,m),8.01(2H,m) |
| 20 | Me | Me | 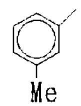 | 5 | $C_{23}H_{29}NO_4$ oil | 1.1-1.8(6H,m),1.9-2.4(4H,m),1.98(6H,s),2.03(3H,s),4.23(1H,t,7Hz),7.05(4H,m) |
| 21 | Me | Me | 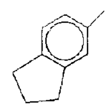 | 6 | $C_{26}H_{33}NO_4$ oil | 1.1-1.8(10H,m),1.9-2.4(4H,m),1.98(6H,s),2.03(3H,s),4.23(1H,t,7Hz),7.05(4H,m) |
| 22 | Me | Me | 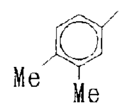 | 5 | $C_{24}H_{31}NO_4$ oil | 1.1-1.8(6H,m),1.9-2.5(4H,m),1.96(6H,s),2.03(3H,s),2.19(6H,s),4.20(1H,t,8Hz),6.98(3H,m) |
| 23 | 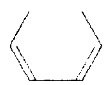 | | 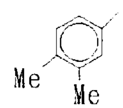 | 5 | $C_{26}H_{29}NO_6$ oil | 1.1-1.8(4H,m),1.9-2.4(4H,m),2.20(3H,s),3.84(6H,s),4.37(1H,t,7Hz),6.83(3H,m),7.66(2H,m),8.02(2H,m) |
| 24 | 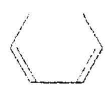 | | 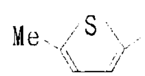 | 5 | $C_{23}H_{24}NO_4S$ oil | 1.1-1.8(4H,m),1.9-2.4(4H,m),2.21(3H,s),2.37(3H,s),4.52(1H,t,7Hz),6.52(1H,d,3Hz),6.67(1H,d,3Hz),7.67(2H,m),8.05(2H,m) |

Table 1 (continued)

| Compound No. | $R^1$ | $R^2$ | $R^3$ | n | molecular formula boiling point (°C) | NMR spectra (in $CDCl_3$), δ(ppm) |
|---|---|---|---|---|---|---|
| 25 | Me | Me | 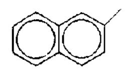 | 5 | $C_{26}H_{29}NO_4$ oil | 1.1-1.8(6H,m),1.81(3H,s),2.0-2.4(4H,m),1.97(3H,s),2.03(3H,s),5.05(1H,t,7Hz),7.2-7.8(7H,m) |
| 26 | Me | Me | 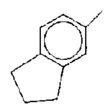 | 5 | $C_{25}H_{31}NO_4$ oil | 1.1-1.8(8H,m),2.96(6H,s),2.01(3H,s),2.83(4H,m),4.21(1H,t,7Hz),7.15(3H,m) |

Example 3

Preparation example

A) Capsules

| | | |
|---|---|---|
| (1) | Compound No.5 | 50 mg |
| (2) | very finely powdered cellulose | 30 mg |
| (3) | lactose | 37 mg |
| (4) | magnesium stearate | 3 mg |
| | total | 120 mg |

Ingredients (1), (2), (3), and (4) were mixed and filled in gelatin capsules.

B) Soft capsules

| | | |
|---|---|---|
| (1) | Compound No.13 | 50 mg |
| (2) | corn oil | 100 mg |
| | total | 150 mg |

C) Tablets

| | | |
|---|---|---|
| (1) | Compound No.6 | 50 mg |
| (2) | lactose | 34 mg |
| (3) | corn starch | 10.6 mg |
| (4) | corn starch (paste) | 5 mg |
| (5) | magnesium stearate | 0.4 mg |
| (6) | calcium carboxymethylcellulose | 20 mg |
| | total | 120 mg |

According to the routine method these ingredients were mixed and compressed by a tableting machine.

Experiment 1 [Inhibition ($10^{-5}$ M) of 5-lipoxygenase from guinea pig polymorphonuclear leukocytes]

5-Lipoxygenase used was the enzyme preparation prepared from guinea pig peritoneal macrophage. For determination of the activity of lypoxygenase 25μM [1-$^{14}$C]arachidonic acid (5 x $10^4$ cpm) as the substrate, 50 mM phosphate buffer solution (pH 7.4), 2 mM $CaCl_2$, 2 mM ATP, and a reaction mixture containing the enzyme (200 μl) were used. After preincubation at 25°C for 2 minutes, [1-$^{14}$C]arachidonic acid (5 x $10^4$ cpm) was added, and allowed to react at 25°C for 3 minutes; the resultant mixture was made acidic, and arachidonic acid and its metabolites were extracted with ether. Radioactivity of the ether layer was determined by silica gel thin layer chromatography using a mixture of petroleum ether:ethylether:acetic acid (15:85:0.1) as the eluant and the elution was performed at -10°C. After elution, the thin layer plate was subjected to autoradiography, the radioactive fractions were scraped out, and the radioactivity of the product was determined by counting. The agent was added two minutes before the beginning of the reaction.

| Compound No. | Inhibitory activity (%) |
|---|---|
| 5 | 74.6 |
| 6 | 79.3 |
| 8 | 83.8 |
| 14 | 89.7 |

Experiment 2 (Inhibition of the binding of U-46619 to platelet membrane fraction)

Blood sampling from guinea pigs and preparation of platelet membrane fraction were performed according to the method of S.C. Hung et al. [Biochim. Biophys. Acta, 728, 171-1.78. (1983)]. Blood was drawn from the heart of a Hartley strain guinea pig anesthetized with ether, and suspended in 3.15% sodium citrate solution (containing aspirin of the final concentration of 1 mM) (sodium citrate solution: whole blood = 1:9). The blood treated with sodium citrate was centrifuged at 3000 rpm for 5 to 6 seconds, so that platelet rich plasma (PRP) was separated. The PRP was again centrifuged at 4°C at 4800 rpm for 10 minutes, to give platelet pellet. The platelet pellet was washed with 30 ml of 25 mM Tris-HCl buffer (containing 5 mM, $MgCl_2$, pH 7.4), and suspended in the same buffer. After platelets were broken by a sonicator, the suspension was centrifuged at 10000 rpm for 1 hour, and the membrane fraction was suspended in the buffer. Determination of protein content was performed by using Biorad protein assay kit, and a suspension containing protein of 1 - 1.5 mg/ml was prepared.

Binding assay was performed as follows: A reaction mixture composed of 4 nM [$^3$H]U-46619, $10^{-9}$ - $10^{-5}$ M drug solution, and platelet membrane fraction containing 100 μg of protein was incubated at 25°C (room temperature) for 30 minutes. The reaction mixture was filtered through a glass filter (GF/C), and washed twice with the buffer described above; the glass filter was placed in 4 ml of liquid scintillator (anionic) for measurement of the radioactivity.

| Compound No. | $IC_{50}$ (M) |
|---|---|
| 8 | 6.0 |
| 14 | 2.6 |

Experiment 3 [Evaluation of inhibition of proliferation of human umbilical venous endothelial cells]

The human vascular endothelial cells were obtained by subculture of the cells, which had been obtained from human umbilical vein by perfusion with tripsin solution, in the culture medium which had been prepared by addition of 2.5% bovine fetal serum and 2.0 ng/ml of human recombinant fibroblast growth factor (abbreviated as rFGF hereinafter; prepared in Biotechnology Institute of our company) to GIT medium (Daigo Eiyou Kagaku).

A hundred μl of the suspension of 2 x $10^3$ human vascular endothelial cells was introduced to a 96-well culture plate (Nunc, 1-67008) and incubated in a gas-controlled thermostat. On the next day rFGF and 100 μl of a medium containing different concentrations of the test substance were added, so that the final concentration of rFGF might be 2 ng/ml. The test substance was dissolved in dimethylsufoxide (DMSO hereinafter) solution, and the resultant solution was diluted so that the final DMSO concentration might be 0.25% or less. After 3 days of incubation, the culture medium containing the test substance was aspirated off, 100 μl of 1 mg/ml MTT solution (3-(4,5-dimethyl-2-thiazolyl)-2,5-diphenyl-2H-tetrazolium bromide dissolved in the culture medium) was added, and the mixture was kept warm for 4 hours. Then 100 μl of 10% SDS solution (aqueous solution of sodium dodecylsulfate) was added and kept warm for 5 - 6 hours, so that the cells and MTT pigment were solubilized, and the $OD_{590}$ value was determined by spectrophotometer. The OD value of the control sample containing no test substance was taken as 100%, and the endothelial cell growth-inhibitory activity of the test substance was compared on the basis of the $IC_{50}$ value, the concentration of a compound at which 50% OD value is observed.

| Compound No. | $IC_{50}$ (μg/ml) |
|---|---|
| 1 | 10 |
| 2 | 0.63 |
| 3 | 1.25 |
| 4 | 0.63 |
| 5 | 0.63 |
| 6 | 1.25 |
| 7 | 1.25 |
| 8 | 0.63 |
| 9 | 1.25 |
| 10 | 25 |
| 11 | 5.0 |
| 12 | 0.08 |
| 13 | < 0.08 |
| 14 | < 0.63 |
| 15 | < 0.63 |
| 16 | 1.25 |
| 17 | 20 |

Experiment 4 [Evaluation of inhibition of growth of IL-2-dependent cells (NKC-3)]

To each well of a 96-well microplate, 50 μl of NKC-3 cells (4 x $10^5$ cells/well), 20 μl of IL-2 solution (0.067 U/ml), and 40 μl of the test substance (DMSO solution) were added, which was incubated at 37°C for 20 hours (culture medium: RPMI1640-20%ECS). To each well 20 μl of MTT solution was added,which was kept at 37°C for 4 hours. Then 100 μl of 10% SDS solution was added to each well, which was kept still at 37°C overnight so that the cells and MTT pigment were solubilized, and the absorbance at 590 nm was measured by spectrophotometer. The absorbance without test substance was taken as 100, and the concentration of a compound that showed 50% absorbance was the $IC_{50}$ value.

| Compound No. | $IC_{50}$ (M) |
|---|---|
| 5 | $4.1 \times 10^{-5}$ |

Experiment 5 [Assay of neo vascularization-inhibiting activity using chicken embryonal chorioallantois]

The procedure of the assay of neo vascularization-inhibiting activity using cultured chicken embryonal chorioallantois was a modification of the method of Taylor et al [S. Taylor & J. Folkman, Nature, 297, 307 (1982)]. Three-day-old fertilized eggs from which the shell had been removed were incubated, and 10 (or 11)-day-old embryos were used. An aqueous solution or an aqueous suspension of the test substance (100 μg) was dried on a transparent plastic disc together with ECGS (endothelial cell growth supplement, Collaborative Research Co.), a vascularizing agent, which was placed on the chorioallantois, and neo vascularization was evaluated after 2 (or 3) days under the stereoscopic microscope by comparing with a control specimen.

| Compound No. | Effectiveness |
|---|---|
| 8 | + |
| 9 | + |
| 14 | + |
| 15 | + |

Experiment 6

Five male ICR mice (8-week-old) in each group were used, and given subcutaneously 100 mg/kg/day of the test substance (Compound No.5) for 3 days. The solution for administration was prepared by dissolving the test substance in physiological saline containing 0.5% gum arabic, and given at the dose of 100 mg/10 ml/kg body weight.

[Results]

During the 4 days of observation after the start of the treatment with the test substance neither death occurred nor abnormality such as loss of body weight was observed.

## Claims

1. Use of a compound of the formula (I) for the manufacture of a pharmaceutical composition for inhibiting cell proliferation in mammals:

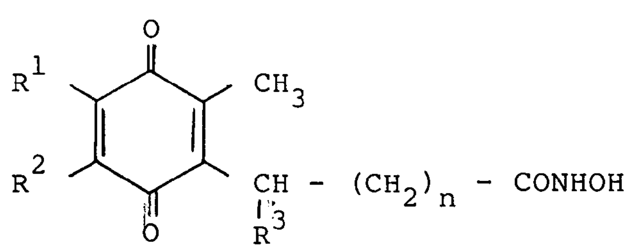

(I)

wherein $R^1$ and $R^2$ are the same or different, representing methyl or methoxy groups, or form -CH=CH-CH=CH- by binding to each other; $R^3$ is an aromatic group or a heterocyclic group (excluding nitrogen-containing heterocyclic groups) which may be substituted; and n is an integer of 2 to 8.

2. Use as claimed in claim 1, wherein n in the formula (I) is an integer of 4 to 6.

3. Use as claimed in claim 1, wherein the aromatic group represented by $R^3$ is phenyl, naphthyl or indanyl, each of which may be substituted with halogen, an alkyl having 1 to 3 carbon atoms, or an alkoxy having 1 to 3 carbon atoms.

4. Use as claimed in claim 1, wherein the heterocyclic group represented by $R^3$ is thienyl or furyl, each of which may be substituted with a halogen, an alkyl having 1 to 3 carbon atoms, or an alkoxy having 1 to 3 carbon atoms.

5. Use as claimed in claim 1, wherein $R^3$ in the formula (I) is a thienyl group which may be substituted with methyl.

6. Use as claimed in claim 1, wherein $R^3$ in the formula (I) is a phenyl group which may be substituted with fluorine or methoxy.

7. A compound of the formula (I'):

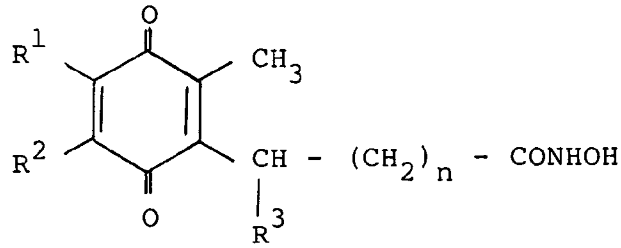

(I')

wherein $R^1$ and $R^2$ are the same or different, representing methyl or methoxy groups, or form -CH=CH-CH=CH- by binding to each other; $R^3$ is an aromatic group or a heterocyclic group (excluding nitrogen-containing heterocyclic groups) which may be substituted; and n is an integer of 2 to 8, but excluding compounds in which $R^1$ and $R^2$ are each methyl, $R^3$ is optionally substituted phenyl and n is 4.

8. A compound as claimed in claim 7, wherein the aromatic group represented by $R^3$ is phenyl or naphthyl, each of which may be substituted with a halogen, an alkyl having 1 to 3 carbon atoms, or an alkoxy having 1 to 3 carbon atoms.

9. A compound as claimed in claim 7, wherein the heterocyclic group represented by $R^3$ is thienyl or furyl, each of which may be substituted with a halogen, an alkyl having 1 to 3 carbon atoms, or an alkoxy having 1 to 3 carbon atoms.

10. A compound as claimed in claim 7, wherein $R^3$ in the formula (I') is a thienyl group which may be substituted with methyl.

11. A compound as claimed in claim 7, wherein $R^3$ in the formula (I') is a phenyl group which may be substituted with fluorine or methoxy.

12. A method for producing a compound of the formula:

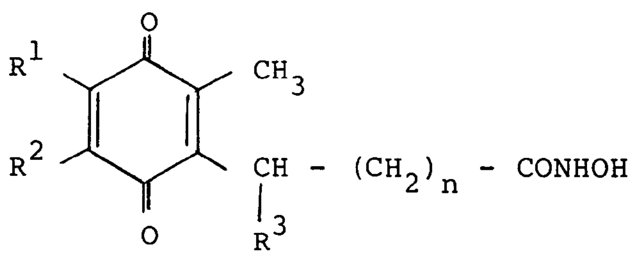

(I')

wherein $R^1$ and $R^2$ are the same or different, representing methyl or methoxy groups, or form -CH=CH-CH=CH- by binding to each other; $R^3$ is an aromatic group or a heterocyclic group (excluding nitrogen-containing heterocyclic groups) which may be substituted; and n is 2 to 8, but excluding compounds in which $R^1$ and $R^2$ are each methyl, $R^3$ is optionally substituted phenyl and n is 4, which comprises reacting a compound of the formula:

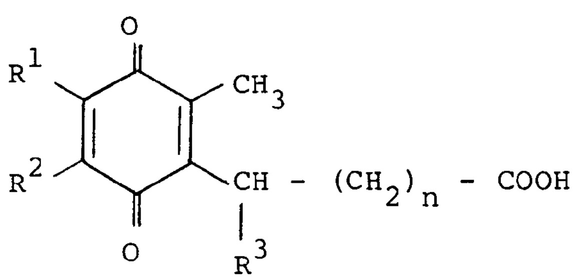

(II)

wherein each symbol has the meaning given above, with a carboxylic acid-activator to give a derivative which is reactive at the carboxylic group, followed by reaction with hydroxylamine.

## Patentansprüche

1. Verwendung einer Verbindung der Formel (I) zur Herstellung einer pharmazeutischen Zusammensetzung zur Hemmung der Zellproliferation in Säugern:

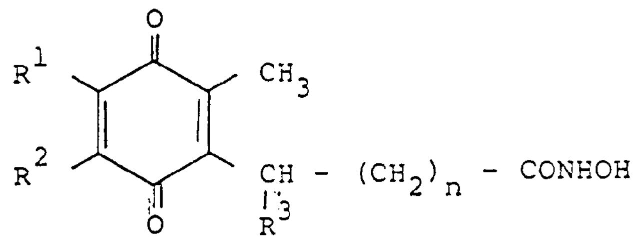

(I)

in der $R^1$ und $R^2$ gleich oder verschieden sind und Methyl- oder Methoxygruppen darstellen oder -CH=CH-CH=CH- durch Verknüpfung miteinander bilden; $R^3$ eine aromatische oder heterocyclische Gruppe (ausgenommen stickstoffhaltige heterocyclische Gruppen) ist, welche substituiert sein kann; und n eine ganze Zahl von 2 bis 8 ist.

2. Verwendung nach Anspruch 1, in der n in der Formel (I) eine ganze Zahl von 4 bis 6 ist.

3. Verwendung nach Anspruch 1, in der die durch $R^3$ dargestellte aromatische Gruppe Phenyl, Naphthyl oder Indanyl ist, welche jeweils mit einem Halogen, einem Alkyl mit 1 bis 3 C-Atomen, oder einem Alkoxy mit 1 bis 3 C-Atomen substituiert sein kann.

4. Verwendung nach Anspruch 1, in der die durch $R^3$ dargestellte heterocyclische Gruppe Thienyl oder Furyl ist, welche jeweils mit einem Halogen, einem Alkyl mit 1 bis 3 C-Atomen, oder einem Alkoxy mit 1 bis 3 C-Atomen substituiert sein kann.

5. Verwendung nach Anspruch 1, in der $R^3$ in der Formel (I) eine Thienylgruppe ist, die mit Methyl substituiert sein kann.

6. Verwendung nach Anspruch 1, in der $R^3$ in der Formel (I) eine Phenylgruppe ist, die mit Fluor oder Methoxy substituiert sein kann.

7. Verbindung der Formel (I'):

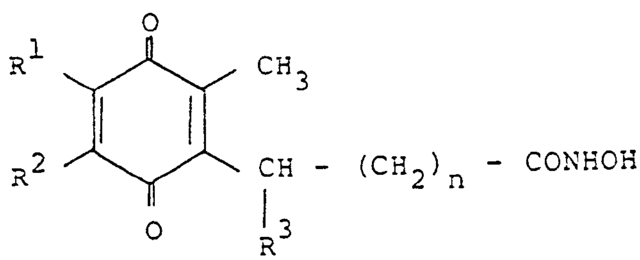

(I')

in der $R^1$ und $R^2$ gleich- oder verschieden sind und Methyl- oder Methoxygruppen darstellen, oder -CH=CH-CH=CH- durch Verknüpfung miteinander bilden; $R^3$ eine aromatische oder heterocyclische Gruppe (ausgenommen stickstoffhaltige heterocyclische Gruppen) ist, welche substituiert sein kann; und n eine ganze Zahl von 2 bis 8 ist, unter Ausschluß von Verbindungen, in denen $R^1$ und $R^2$ beide Methyl sind, $R^3$ gegebenenfalls substituiertes Phenyl ist und n 4 ist.

8. Verbindung nach Anspruch 7, in der die durch $R^3$ dargestellte aromatische Gruppe Phenyl oder Naphthyl ist, welche jeweils mit einem Halogen, einem Alkyl, mit 1 bis 3 C-Atomen, oder ein Alkoxy mit 1 bis 3 C-Atomen substituiert sein kann.

9. Verbindung nach Anspruch 7, in der die durch $R^3$ dargestellte heterocyclische Gruppe Thienyl oder Furyl ist, welche jeweils mit einem Halogen, einem Alkyl mit 1 bis 3 C-Atomen oder einem Alkoxy mit 1 bis 3 C-Atomen substituiert sein kann.

10. Verbindung nach Anspruch 7, in der $R^3$ in der Formel (I') eine Thienylgruppe ist, die mit Methyl substituiert sein kann.

11. Verbindung nach Anspruch 7, in der $R^3$ in der Formel (I') eine Phenylgruppe ist, die mit Fluor oder Methoxy substituiert sein kann.

12. Verfahren zur Herstellung einer Verbindung der Formel:

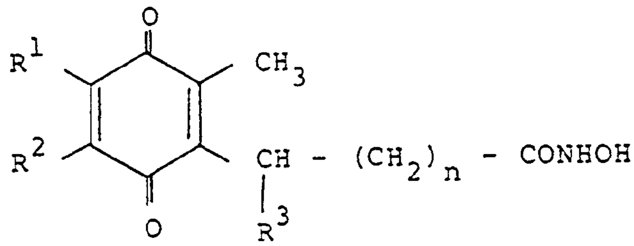

(I')

in der $R^1$ und $R^2$ gleich- oder verschieden sind und Methyl- oder Methoxygruppen darstellen oder -CH=CH-CH=CH- durch Verknüpfung miteinander bilden; $R^3$ eine aromatische oder heterocyclische Gruppe (ausgenommen stickstoffhaltige heterocyclische Gruppen) ist, welche substituiert sein kann; und n 2 bis 8 ist, unter Ausschluß von Verbindungen, in denen $R^1$ und $R^2$ beide Methyl sind, $R^3$ gegebenenfalls substituiertes Phenyl und n 4 ist, umfassend die Umsetzung einer Verbindung der Formel:

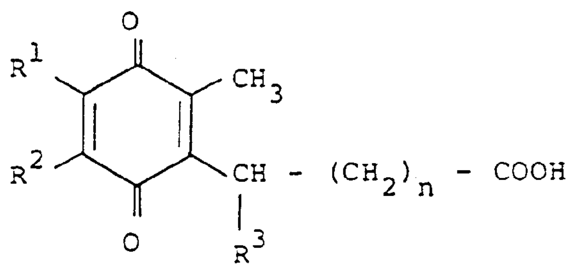

(II)

in der jedes Symbol die oben angegebene Bedeutung hat, mit einem Carbonsäure-Aktivator zum Erhalt eines Derivats, das an der Carbonsäuregruppe reaktionsfähig ist, gefolgt von der Umsetzung mit Hydroxylamin.

**Revendications**

1. Utilisation d'un composé de formule (I) pour la fabrication d'une composition pharmaceutique destinée à inhiber la prolifération cellulaire chez les mammifères:

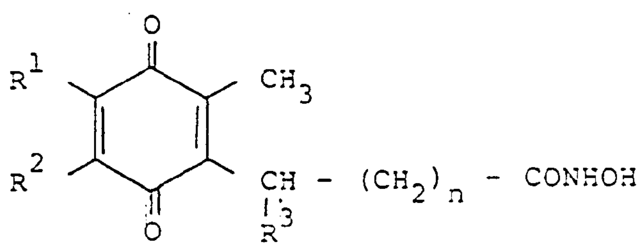

(I)

formule dans laquelle $R^1$ et $R^2$ sont identiques ou différents et représentent des groupes méthyle ou méthoxy ou forment par liaison de l'un avec l'autre un groupe -CH=CH-CH=CH-, $R^3$ représente un groupe aromatique ou un groupe hétérocyclique (à l'exclusion de groupes hétérocycliques contenant de l'azote) qui peut être substitué et n représente un nombre entier de 2 à 8.

2. Utilisation selon la revendication 1, dans laquelle n dans la formule (I) est un nombre entier de 4 à 6.

3. Utilisation selon la revendication 1, dans laquelle le groupe aromatique représenté par $R^3$ est un phényle, un naphthyle ou un indanyle qui peuvent être substitués chacun par un halogène, un alcoyle comportant de 1 à 3 atomes de carbone ou un alcoxy comportant de 1 à 3 atomes de carbone.

4. Utilisation selon la revendication 1, dans laquelle le groupe hétérocyclique représenté par $R^3$ est un thiényle ou un furyle qui peuvent être substitués chacun par un halogène, un alcoyle comportant de 1 à 3 atomes de carbone ou un alcoxy comportant de 1 à 3 atomes de carbone.

5. Utilisation selon la revendication 1, dans laquelle $R^3$ dans la formule (I) est un groupe thiényle qui peut être substitué par un méthyle.

6. Utilisation selon la revendication 1, dans laquelle $R^3$ dans la formule (I) est un groupe phényle qui peut

être substitué par un fluor ou un méthoxy.

7. Composition de formule (I'):

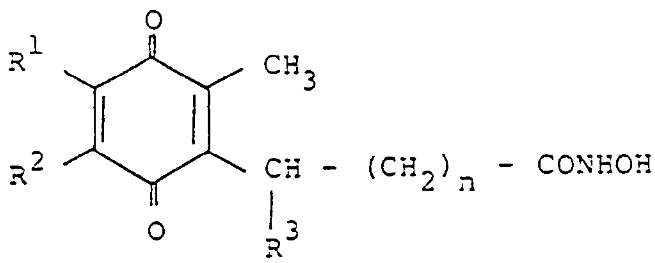

(I')

dans laquelle $R^1$ et $R^2$ sont identiques ou différents et représentent des groupes méthyle ou méthoxy ou forment par liaison de l'un avec l'autre un groupe -CH=CH-CH=CH-, $R^3$ représente un groupe aromatique ou un groupe hétérocyclique (à l'exclusion des groupes hétérocycliques contenant de l'azote) qui peut être substitué et n représente un nombre entier de 2 à 8, mais à l'exception des composés dans lesquels $R^1$ et $R^2$ sont chacun un méthyle, $R^3$ est un phényle éventuellement substitué et n est 4.

8. Composé selon la revendication 7, dans lequel le groupe aromatique représenté par $R^3$ est un phényle ou un naphtyle qui peuvent être substitués chacun par un halogène, un alcoyle comportant de 1 à 3 atomes de carbone ou un alcoxy comportant de 1 à 3 atomes de carbone.

9. Composé selon la revendication 7, dans lequel le groupe hétérocyclique représenté par $R^3$ est un thiényle ou un furyle qui peuvent être substitués chacun par un halogène, un alcoyle comportant de 1 à 3 atomes de carbone ou un alcoxy comportant de 1 à 3 atomes de carbone.

10. Composé selon la revendication 7, dans lequel $R^3$ dans la formule (I') est un groupe thiényle qui peut être substitué par un méthyle.

11. Composé selon la revendication 7, dans lequel $R^3$ dans la formule (I') est un groupe phényle qui peut être substitué par un fluor ou un méthoxy.

12. Procédé de préparation d'un composé de formule:

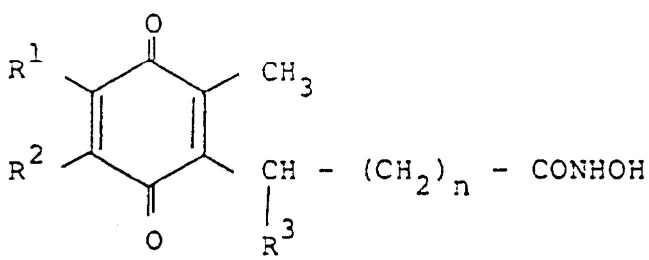

(I')

dans laquelle $R^1$ et $R^2$ sont identiques ou différents et représentent des groupes méthyle ou méthoxy ou forment par liaison de l'un avec l'autre un groupe -CH=CH-CH=CH-, $R^3$ représente un groupe aromatique ou un groupe hétérocyclique (à l'exclusion des groupes hétérocycliques contenant de l'azote) qui peut être substitué et n représente un nombre entier de 2 à 8, mais à l'exception des composés dans lesquels $R^1$ et $R^2$ sont chacun un méthyle, $R^3$ est un phényle éventuellement substitué et n est 4, selon lequel on fait réagir un composé de formule:

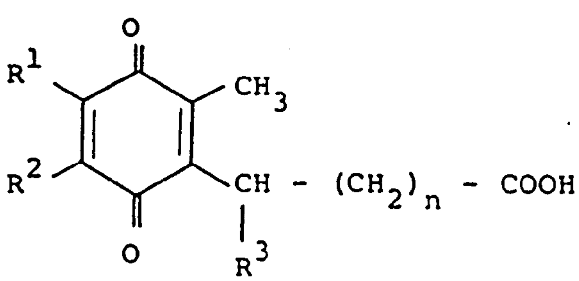

(II)

dans laquelle chaque symbole a la signification indiquée ci-dessus, avec un agent d'activation d'acide carboxylique, pour former un dérivé réactif au groupe carboxylique, puis on fait réagir avec l'hydroxylamine.